# EUROPÄISCHE PATENTSCHRIFT

(11) **EP 1 987 864 B1**
(45) Veröffentlichungstag und Bekanntmachung des Hinweises auf die Patenterteilung: **17.02.2010**
(21) Anmeldenummer: 08005262.4
(22) Anmeldetag: 20.03.2008
(51) Int. Cl.: A63B 23/18

(54) **Therapiegerät**
Therapy device
Appareil de thérapie

(30) Priorität: 16.04.2007 DE 102007017783
(43) Veröffentlichungstag der Anmeldung: 05.11.2008
(73) Patentinhaber: R. Cegla GmbH & Co. KG, 56410 Montabaur (DE)
(72) Erfinder: Cegla, Ulrich Prof. Dr. med., 56510 Montabaur (DE)
(74) Vertreter: Engelhardt, Volker

(56) Entgegenhaltungen:
- EP-A- 1 772 165
- DE-A1- 4 416 575
- FR-A- 1 016 678

## Beschreibung

Die Erfindung bezieht sich auf ein Therapiegerät zur Unterstützung der Atmung, der Schleimlösung und der Expektoration eines Patienten, mittels dem beim Aus- und Einatmen im Luftstrom ein oszillierender Luftwiderstand erzeugbar ist, mit einem Mundstück, an dessen einer Stirnseite eine Durchlassöffnung vorgesehen ist und an dessen der Durchlassöffnung gegenüberliegenden Stirnseite ein elastisch verformbares Schlauchstück befestigt ist, bzw. die Erfindung betrifft ein Therapiegerät, bestehend aus einem Mundstück an dessen einer Stirnseite eine Durchlassöffnung vorgesehen und an dessen der Durchlassöffnung gegenüberliegenden Stirnseite ein elastisch verformbares Schlauchstück befestigt ist und das aus einem Gehäuse besteht, das fest mit dem Mundstück verbunden und durch das das Schlauchstück in Umfangsrichtung ganz oder teilweise ummantelt ist.

Ein solches Therapiegerät kann beispielsweise der DE 4416575 entnommen werden, das im Wesentlichen aus einem viertelkreisförmig gekrümmten Gehäuse besteht, in das das Mundstück eingeschraubt werden kann. Am Austritt des Mundstückes ist das Schlauchstück befestigt, das somit auf der Innenseite des gekrümmten Gehäuses aufliegt, wobei das Schlauchstück durch die Krümmung des Gehäuses abgewinkelt wird. Durch Verdrehen des Mundstücks im Gehäuse wird der Abschnitt des Schlauchstückes, der an dem Gehäuse anliegt, vergrößert oder verkleinert in Abhängigkeit von der Drehrichtung des Mundstückes. Wenn demnach das Mundstück in Richtung des Gehäuses eingeschraubt wird, vergrößert sich der Abschnitt des Schlauchstückes, der an der Innenwand des Gehäuses anliegt, und umgekehrt.

Beim Ein- und Ausatmen durch das Mundstück entsteht ein Luftstrom, der somit vom Patienten eingesogen oder ausgeblasen wird. Aufgrund der Querschnittsfläche des Schlauchstückes, die kleiner bemessen ist als die Querschnittsfläche der Durchgangsöffnung durch das Mundstück, entsteht bereits ein Luftwiderstand, durch den die menschliche Atmung aktiviert wird. Dadurch soll die Lungenperipherie bei Erkrankungen, beispielsweise bei chronischer Bronchitis, Bronchiektasen, Mukoviszidose, Lungenemphysem und dgl. behandelt werden können.

Als nachteilig bei dem bekannten Therapiegerät hat sich herausgestellt, dass die Einstellungen des durch das Schlauchstück hervorgerufenen Luftwiderstandes ausschließlich im unbetätigten Zustand vorgenommen werden kann, denn der Patient hat, um das Mundstück verstellen zu können, dieses abzusetzen.

Des Weiteren ist nachteilig, dass die Einstellung nur unwesentlich dazu beiträgt, den Luftwiderstand zu reduzieren oder zu erhöhen, denn aufgrund der vorgegebenen Krümmung des Gehäuses des Therapiegerätes wird eine wesentliche Winkeländerung bzw. der Krümmung des Schlauchstückes nicht erreicht. Zur Behandlung von Atmungsproblemen ist es jedoch erforderlich, die Druckamplituden und die niedrigeren Schwingungsfrequenzen einstellen zu können, um den in der Lunge zu lösenden Schleim aus dieser durch die Atmung hinauszubefördern. Hierzu sind Frequenzänderungen und Druckunterschiede erforderlich, um die Druck- und Volumentstromschwankung während des Atemvorganges anzuregen.

Die Einstellung der Resonanzfrequenz, die der Brustkorb zur Überwindung des Luftwiderstandes beim Ein- und Aufatmen aufweisen sollte, liegt erfahrungsgemäß zwischen 12 und 14 Hertz, denn bei dieser Frequenz wird der Arbeitsaufwand für die Aus- und Einatmung verringert, wodurch das Therapiegerät eine länger bemessene Zeitdauer verwendet werden kann. Eine solche therapeutische Handhabung des bekannten Therapiegerätes ist jedoch nicht möglich, da die Einstellung der Resonanzfrequenz während des Ein- und Ausatmens nicht verändert werden kann, so dass der Patient gezwungen ist, die Einstellungen während der Atempausen vorzunehmen, wodurch eine exakte und auf jeden Patienten individuell abgestimmte Einstellung nicht möglich ist und auch die Frequenzänderung während der Atmung nicht vorgenommen werden können.

Darüber hinaus weist die Ausbildung des bekannt gewordenen Therapiegeräts den Nachteil auf, dass durch die fest vorgegebene Krümmung der Gehäusewand lediglich eine bestimmte eng bemessene Bandbreite von Einstellungsmöglichkeiten besteht, durch die der Luftwiderstand zu verändern ist.

Es ist daher Aufgabe der Erfindung, ein Therapiegerät der eingangs genannten Gattung bereit zu stellen, das zur Behandlung von Atembeschwerden wie Mukoviszidose, Bronchiektasie, Raucherlungen und dgl. einfach zu handhaben ist und bei dem die Atem-Frequenzen und die Druckamplituden während des Ein- und Ausatmens individuell an die Patientengegebenheit in einen möglichst groß bemessenen Anwendungsbereich angepasst werden können.

Diese Aufgabe wird erfindungsgemäß dadurch gelöst, dass an dem Mundstück eine Auflageplatte angebracht ist, die im Bereich des Mundstückes parallel zu dem Schlauchstück verlaufend ausgerichtet ist, dass an dem freien Ende der Auflageplatte ein Drehgelenk vorgesehen ist, über das ein Widerstandskörper an der Auflageplatte angelenkt ist, und dass der Widerstandskörper mit dem diesem zugeordneten Bereich des Schlauchstückes derart zusammenwirkt, dass der Querschnitt des Schlauchstückes von außen variabel einstellbar ist, oder dass beanstandet zu dem Mundstück im Inneren des Gehäuses ein an diesem bewegbar angelenkten Widerstandskörper angeordnet ist, dass der Widerstandskörper als Auflager für einen Teil des freien Endes des Schlauchstückes vorgesehen ist und dass durch das Auflager die Krümmung des Schlauchstückes einstellbar ist.

Weitere vorteilhafte Weiterbildungen der Erfindung ergeben sich aus den Unteransprüchen.

Dadurch, dass das Widerlager von außen zugänglich und über das Drehgelenk oder über die Verrastungselemente in einer bestimmten Winkelposition überführbar ist, kann die Krümmung des Schlauchstückes in einem großen Winkelbereich eingestellt werden, so dass der im Schlauchstück anstehende Luftstrom an die Resonanzfrequenz des Brustkorbes des Patienten angepasst werden kann. Somit kann während der Ein- und Ausatmung durch den Patienten die Einstellung für die Resonanzfrequenz und die Druckamplitude vorgenommen werden.

Wenn die optimale Einstellungsposition des Widerstandskörpers gefunden wurde, erhöht sich die Zeitdauer, innerhalb der der Patient das Therapiegerät verwenden kann, denn durch die eingestellte Resonanzfrequenz wird das Ausatmen erleichtert, wodurch der Patient das Therapiegerät über einen längeren Zeitraum verwenden kann ohne dass der Behandlungserfolg beeinträchtigt ist. Dies wiederum führt dazu, dass die Behandlungserfolge in einem wesentlich kürzer bemessenen Zeitraum erreicht werden, denn der Patient wird das erfindungsgemäße Therapiegerät öfter und länger verwenden.

In der Zeichnung sind drei erfindungsgemäße Ausführungsbeispiele dargestellt, die nachfolgend näher erläutert werden. Im Einzelnen zeigt:
- Figur 1a: ein erstes Ausführungsbeispiel eines Therapiegerätes, das aus einem Mundstück und einem Schlauchstück besteht, die an einer Auflageplatte befestigt sind, an dessen freien Ende ein Drehgelenk zur Bewegung eines Widerstandskörpers vorgesehen ist, in perspektivischer Ansicht,
- Figur 1b: das Ausführungsbeispiel des Therapiegerätes gemäß der Fig. 1 a im angehobenen Zustand des Widerstandskörpers,
- Figur 1c: das Terapiegerät gemäß Figur 1b, im Schnitt,
- Figur 2: ein zweites Ausführungsbeispiel, in perspektivischer Ansicht und
- Figur 3: ein drittes Ausführungsbeispiel eines Therapiegerätes, das aus einem Mundstück, an dessen Ende ein Schlauchstück befestigt ist, und aus einem Gehäuse besteht, durch das das Mundstück gehalten ist und durch das das Schlauchstück vollständig ummantelt ist und in dessen Inneren ein Widerstandskörper verschwenkbar angelenkt ist, mit einem vollständig geschlossenen Gehäuse, in perspektivischer Ansicht.

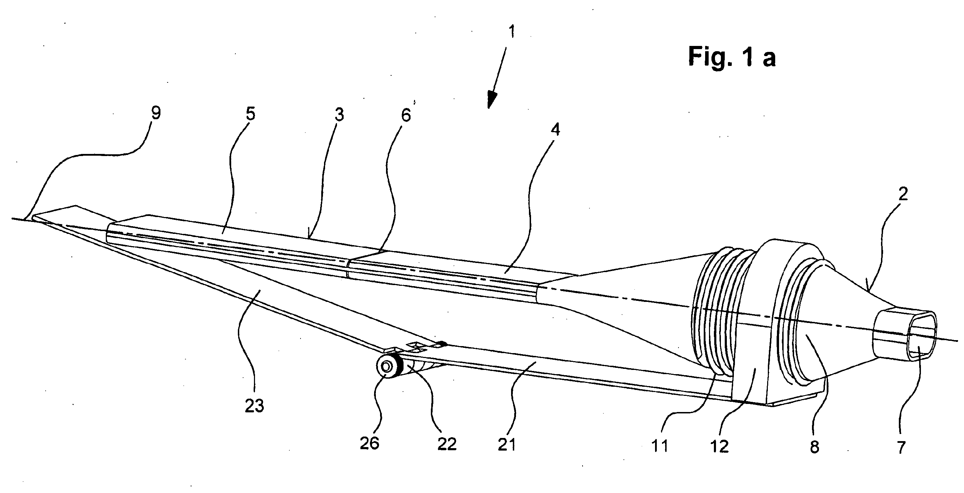

In den Figuren 1a, 1b, 1c, 2 und 3 ist ein Therapiegerät 1 dargestellt, das zur Unterstützung der Atmung, der Schleimlösung und der Expektoration eines Patienten dient. Das Therapiegerät 1 besteht aus einem Mundstück 2, an dem eine Einlassöffnung 7 vorgesehen ist, die beispielsweise in Nasen- oder Mundöffnungen eines Patienten einschiebbar ist. Im Inneren des Mundstücks 2 ist ein Durchgangskanal 8 eingearbeitet, der in die Einlassöffnung 7 mündet.

Gegenüberliegend zu der Stirnseite, in der die Einlassöffnung 7 vorgesehen ist, ist ein Schlauchstück 3 fest mit dem Mundstück 2 verbunden. Die Querschnittsform des Schlauchstückes 3 ist dabei rechteckförmig ausgebildet. Der Durchgangskanal 8 verjüngt sich in Richtung des Schlauchstückes 3, so dass die durch die Einlassöffnung 7 strömende Atmungsluft durch den Kanal 7 und das Schlauchstück 3 gepresst werden muss. Somit entsteht insbesondere im Schlauchstück 3 ein Luftstrom, durch den das freie Ende des Schlauchstückes 3 bewegt wird, wodurch ein oszillierender Luftwiderstand für den Luftstrom durch das Schlauchstück 3 in Abhängigkeit von dessen Länge und Querschnittsfläche entsteht.

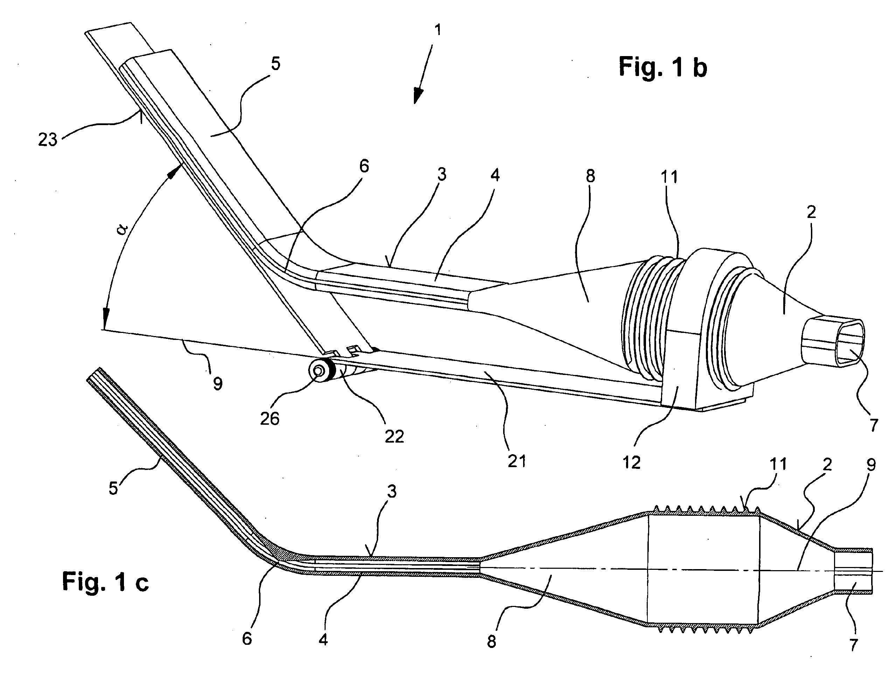

Um den oszillierenden Luftwiderstand an die individuellen Eigenschaften des Patienten anpassen zu können, nämlich um die Resonanzfrequenz des Brustkorbes beim Ein- und Ausatmen zu erreichen, ist das erfindungsgemäße Therapiegerät 1 gemäß den Figuren 1a, 1b und 1c mit einer Auflageplatte 21 ausgestattet, die über ein Haltering 12 fest mit dem Mundstück 2 verbunden ist. Die Auflageplatte 21 verläuft dabei parallel zu einem ersten Abschnitt 4 des Schlauchstückes 3. An dem freien Ende der Auflageplatte 21 ist ein Drehgelenk 22 angebracht, durch das ein Widerstandskörper 23 verschwenkbar gehalten ist. Der Widerstandskörper 23 wirkt mit einem zweiten Abschnitt 5 des Schlauchstückes 3 zusammen.

Insbesondere der Figur1a kann entnommen werden, dass der Widerstandskörper 23 als Auflager für den zweiten Abschnitt 5 des Schlauchstückes 3 dient, denn das Schlauchstück 3 liegt auf dem Widerstandskörper 23 auf, so dass die beiden Abschnitte 4 und 5 des Schlauchstückes 3 in dieser Einstellungsposition fluchtend zu der Längsachse 9 des Therapiegerätes 1 verlaufen. Die Querschnittsfläche des Schlauchstückes 3 wird demnach über dessen gesamten Länge nicht verändert. Beim Ein- und Ausatmen des Patienten strömt daher die Atemluft durch das Innere des Schlauchstückes 3 ungehindert hindurch. Das freie Ende des Schlauchstückes 3 flattert auf dem Widerstandskörper 23 und wird durch diesen in seiner Bewegung einseitig begrenzt.

Falls die derart erzeugte Oszillation der Atemluft die Resonanzfrequenz für den jeweiligen Patienten nicht erreicht, kann der Widerstandskörper 23 in eine bestimmte Winkellage, die zwischen 0° - dies entspricht der gezeigten Stellung in Figur 1a - und einer Auslenkung von 50° bewegt werden. Durch die Winkeländerung des Widerstandskörpers 23 in Bezug auf die Längsachse 9 des Therapiegerätes 1 wird der zweite Abschnitt 5 des Schlauchstückes 3, wie dies in den Figuren 1b und 1c gezeigt ist, aus der Längsachse 9 angehoben, so dass zwischen den beiden Abschnitten 4 und 5 des Schlauchstückes 3 ein Krümmungs- oder Knickbereich 6 ausgebildet ist. Die über die gesamte Länge des Schlauchstückes 3 konstante Querschnittsfläche wird demnach im Bereich der Knickung 6 in Abhängigkeit von der gewählten Winkelstellung des Widerstandskörpers 23 verkleinert. Dies bedeutet, wenn der Widerstandskörper 23 aus der 0°-Stellung in die 50°-Stellung überführt wird, weist die Querschnittsfläche in der 0°-Stellung die größte Durchtrittsfläche und in der 50°-Stellung die kleinste Durchtrittsfläche auf.

Der Patient kann demnach während der Ein- und Ausatmung die Position bzw. die Winkelstellung des Widerstandskörpers 23 individuell anpassen, so dass die gewünschte Resonanzfrequenz während der Atmung einstellbar ist. Das Drehgelenk 22 ist dabei derart ausgebildet, dass dieses durch eine Feststellschraube 26 festgesetzt werden kann, so dass die Position des Widerstandskörpers 23 in der ausgewählten Winkellage fixiert ist.

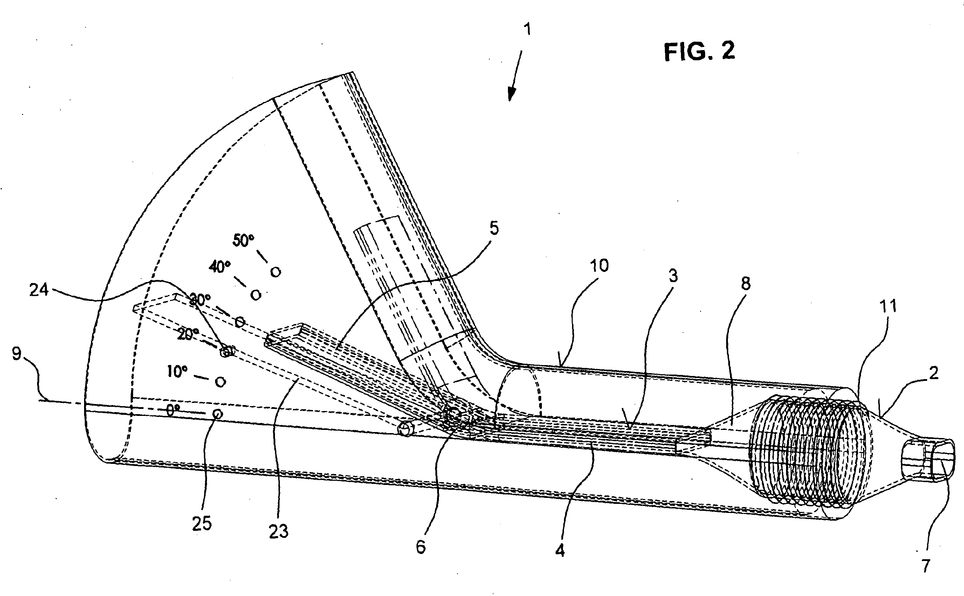

In Figur 2 ist das Mundstück 2 in ein Gehäuse 10 eingeschraubt, so dass das Gehäuse 10 das Schlauchstück 3 vollständig ummantelt. Die Kontur des Gehäuses 10 kann dabei in zwei Abschnitte unterteilt werden, nämlich in einen runden ersten Abschnitt, der sich parallel zu dem ersten Abschnitt 4 des Schlauchstückes 3 erstreckt und einem zweiten Abschnitt, der dem zweiten Abschnitt 5 des Schlauchstückes 3 zugeordnet ist.

Der zweite Abschnitt des Gehäuses 10 soll dabei trompeten- oder V-förmig ausgebildet sein, so dass das freie Ende des Schlauchstückes 3 zwischen dem Widerstandskörper 23, der drehbar an einer oder an beiden Seitenwänden des Gehäuses 10 abgestützt ist, und der dem Widerstandskörper 23 gegenüberliegenden Innenwand des Gehäuses 10 bewegbar ist. Die Stirnseite des Gehäuses 10 ist dabei vollständig offen.

Wenn demnach die Winkelstellung des Widerstandskörpers 23 in Richtung der Innenwand des Gehäuses 10 bewegt wird, zwischen denen das Schlauchstück 3 angeordnet ist, verringert sich die Bewegungsmöglichkeit des freien Endes des Schlauchstückes 3 derart, dass dieses zwischen dem Widerstandskörper 23 und der darüber liegenden Innenwand des Gehäuses 10 hin und her flattert. Die Atemluft strömt aus der Stirnseite des Gehäuses 10 ins Freie.

Der Widerstandskörper 23 weist eine in Richtung der Seitenwand des Gehäuses 10 abstehende Feder-Rastnase 23 auf. In dieser Seitenwand des Gehäuses 10 sind mehrere Bohrungen 25 eingearbeitet, in die die Rastnase 24 einschnappt, um die Position des Widerstandskörpers 23 in einer bestimmten Winkelstellung zu arretieren.

Eine weitere Einstellungsmöglichkeit, um die Resonanzfrequenz des Therapiegerätes 1 zu erreichen, besteht darin, dass das Mundstück 2 relativ zu dem Widerstandskörper 23 in Längsrichtung 9 verschoben wird. Bei dem Ausführungsbeispiel nach den Figuren 1a, 1b und 1c wird das Mundstück 2 in dem Haltering 12 fixiert und in dem Ausführungsbeispiel der Figur 2 ist das Mundstück 2 mit dem Gehäuse 10 verschraubt. Durch die Positionsveränderung des Mundstückes 2 wird demnach entweder mehr oder weniger des zweiten Abschnittes 5 des Schlauchstückes 3 auf den Widerstandskörper 23 angeordnet, wodurch ebenfalls die Resonanzfrequenz des Schlauchstückes 3 verändert werden kann. Ferner führt die Verdrehung des Mundstückes 2 dazu, dass das Schlauchstück 3 in unterschiedliche Positionen auf dem Widerstandskörper 23 aufliegt, wodurch ebenfalls eine Frequenzänderung des Luftstroms erreicht werden kann.

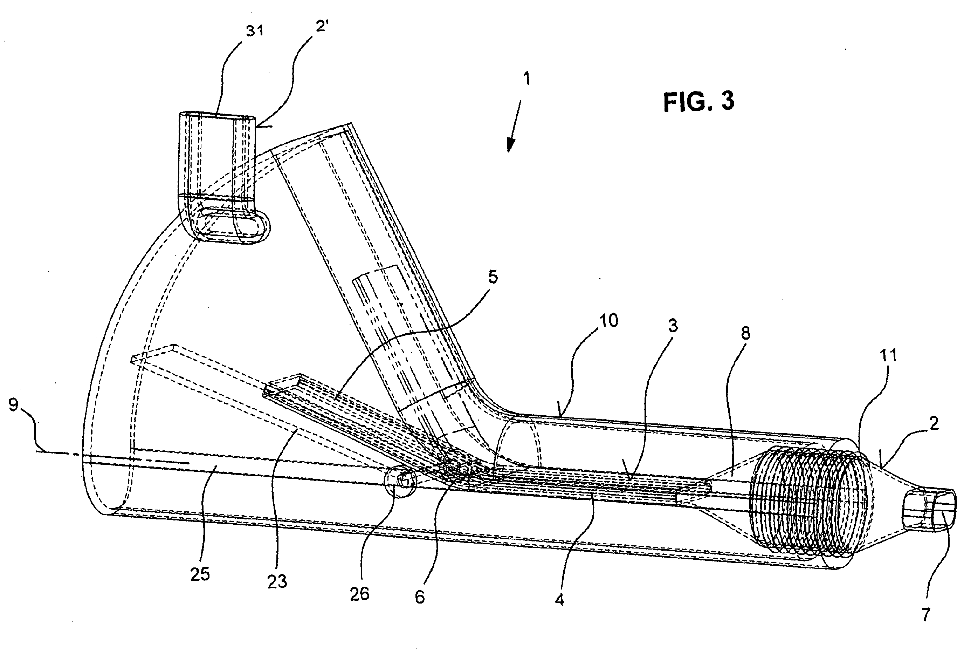

In Figur 3 ist das Gehäuse 10 vollständig geschlossen. In einer Seitenwand des Gehäuses 10 ist eine Öffnung 31 eingearbeitet, in die ein zweites Mundstück 2' eingesetzt ist, durch das Luft aus dem Inneren des Gehäuses 10 herausgesogen werden kann. Im Inneren des Gehäuses 10 entsteht demnach beim Einatmen ein Unterdruck, durch den ein Luftstrom durch das Schlauchstück 3 und das erste Mundstück 2 gebildet wird. Dieser Luftstrom bewirkt ein Flattern des Schluchstückes 3, so dass hierbei ein oszilierender Luftwiderstand bei der Einatmung entsteht.

Die Feststellschraube 26 ist außerhalb des Gehäuses 10 angeordnet. Durch diese ist sowohl die Verstellung als auch die Arretierung des Widerstandskörpers 23 gegeben.

## Patentansprüche

1. Therapiegerät (1) zur Unterstützung der Atmung, der Schleimlösung und der Expektoration eines Patienten, mittels dem beim Aus- und Einatmen im Luftstrom ein oszillierender Luftwiderstand erzeugbar ist, mit einem Mundstück (2), an dessen einer Stirnseite eine Einlassöffnung (7) vorgesehen und an dessen der Einlassöffnung gegenüberliegender Stirnseite ein elastisch verformbares Schlauchstück (3) befestigt ist,
**dadurch gekennzeichnet,**
**dass** an dem Mündstück (2) eine Auflageplatte (21) angebracht ist, die im Bereich des Mundstückes (2) parallel zu dem Schlauchstück (3) verlaufend ausgerichtet ist, dass an dem freien Ende der Auflageplatte (21) ein Drehgelenk (22) vorgesehen ist, über das ein Widerstandskörper (23) an der Auflageplatte (21) angelenkt ist, und dass der Widerstandskörper (23) mit dem diesen zugeordneten Abschnitt (5) des Schlauchstückes (3) derart zusammen wirkt, dass die Durchlassfläche des Schlauchstück (3) von außen variabel einstellbar ist.

2. Therapiegerät nach Anspruch 1,
**dadurch gekennzeichnet,**
**dass** der Widerstandskörper (23) in Richtung des Schlauchstückes (3) in einem Winkelbereich von 0° bis 50° bezogen auf die Längsachse (9) des Therapiegerätes (1) auslenkbar ist.

3. Therapiegerät nach Anspruch 1 oder 2,
**dadurch gekennzeichnet,**
**dass** das Mundstück (2) in ein Gehäuse (10) eingesetzt ist, durch das das Schlauchstück (3) in Umfangsrichtung umschlossen ist, dass das dem Mundstück (2) gegenüberliegende Ende des Gehäuses (10) eine trompeten- oder V-förmig ausgebildete Durchgangsöffnung aufweist und dass der Widerstandskörper (23) im Bereich der Durchgangsöffnung angeordnet ist.

4. Therapiegerät nach Anspruch 3,
**dadurch gekennzeichnet,**
**dass** an dem Widerstandskörper (23) eine Rastnase (24), ein Federelement od. dgl. vorgesehen ist, die in Richtung einer der beiden Seitenwände des Gehäuses (10) abstehen, und dass in die Gehäusewand eine oder mehrere Bohrungen (25) eingearbeitet sind, in die die Rastnase (24) oder das Federelement zur Feststellung des Widerstandskörper (23) in einer bestimmten Winkelstellung einführbar ist.

5. Therapiegerät (1) zur Unterstützung der Atmung und der Expektoration eines Patienten, mittels dem beim Ein- und Ausatmen im Luftstrom ein oszillierender Luftwiderstand erzeugbar ist, bestehend aus einem Mundstück (2), an dessen einer Stirnseite eine Einlassöffnung (7) vorgesehen und an dessen der Einlassöffnung (7) gegenüberliegender Stirnseite ein elastisch verformbares Schlauchstück (3) befestigt ist, und aus einem Gehäuse (10), das fest mit dem Mundstück (2) verbunden und durch das das Schlauchstück (3) in Umfangsrichtung ganz oder teilweise ummantelt ist,
**dadurch gekennzeichnet,**
**dass** beabstandet zu dem Mundstück (2) im Inneren des Gehäuses (10) ein an diesem bewegbar angelenkter Widerstandskörper (23) angeordnet ist, dass der Widerstandskörper (23) als Auflager für einen Abschnitt (5) des Schlauchstückes (3) vorgesehen ist, und dass durch das Auflager die Krümmung des Schlauchstückes (3) einstellbar ist.

6. Therapiegerät nach Anspruch 5,
**dadurch gekennzeichnet,**
**dass** das Schlauchstück (3), vorzugsweise das freie Ende des Schlauchstückes (3), zwischen dem Widerstandskörper (23) und der diesem gegenüberliegenden Innenwand des Gehäuses (10) beim Ein- und Ausatmen oszillierend bewegbar ist.

7. Therapiegerät nach einem oder mehreren der vorgenannten Ansprüche,
**dadurch gekennzeichnet,**
**dass** das Mundstück (2) zusammen mit dem Schlauchstück (3) in Bezug auf den Widerstandskörper (23) in Längsachse (9) relativ zu diesem beweglich ausrichtbar ist.

8. Therapiegerät nach einem oder mehreren der vorgenannten Ansprüche,
**dadurch gekennzeichnet,**
**dass** der Widerstandskörper (23) als Platte ausgebildet ist und dass ein Abschnitt (5) des Schlauchstückes (3) von dem Widerstandkörpers (23) im unbetätigten Zustand in ein Höhenniveau überführbar ist, das unter- oder oberhalb des von den an dem Mundstückes (2) befestigten Abschnittes (4) des Schlauchstückes (3) liegt.

9. Therapiegerät nach einem oder mehreren der vorgenannten Ansprüche,
**dadurch gekennzeichnet,**
**dass** das Schlauchstück (3) in unterschiedliche Winkelpositionen bezüglich des Widerstandskörpers (23) ausrichtbar ist.

10. Therapiegerät nach Anspruch 5,
**dadurch gekennzeichnet,**
**dass** das Gehäuse (10) vollständig geschlossen ausgebildet ist, dass in eine der Seitenwände des Gehäuses (10) eine Öffnung (31) eingearbeitet ist und dass in die Öffnung (31) ein zweites Mundstück (2') eingesetzt ist.

## Claims

1. A therapy device (1) for supporting the respiration, mucolytic action and expectoration of a patient by means of which an oscillating air resistance can be produced in the air flow during exhalation and inhalation, with a mouthpiece (2) having an inlet opening (7) provided on one of its ends and an elastically deformable hose section (3) attached to its end that is opposite to the inlet opening (7),
**characterised in that,**
the mouthpiece (2) has a contact plate (21) attached to it that is aligned running in parallel with the hose section (3) in the area of the mouthpiece (2), that the free end of the contact plate (21) has a swivel joint (22) provided on it, by means of which a resistance body (23) is articulated on the contact plate (21), and that the resistance body (23) interacts with the section (5) of the hose section (3) that is assigned to them, in such a way that the passage cross-sectional area of the hose section (3) can be variably adjusted.

2. The therapy device in accordance with Claim 1,
**characterised in that,**
the resistance body (23) in the direction of the hose section (3) can be deflected in an angle range from 0° to 50° in relation to the longitudinal axis (9) of the therapy device (1).

3. The therapy device in accordance with Claim 1 or 2,
**characterised in that,**
the mouthpiece (2) is inserted in a housing (10) by means of which the hose section (3) is enclosed in the circumferential direction, that the end of the housing (10) opposite to the mouthpiece (2) has a trumpet-shaped or V-shaped passage opening and that the resistance body (23) is arranged in the area of the passage opening.

4. The therapy device in accordance with Claim 3,
**characterised in that,**
the resistance body (23) has a detent lug (24), a spring element or the like provided on it, which protrude in the direction of one of the two side walls of the housing (10), and that that housing wall has one or more holes (25) worked into it, into which the detent lug (24) or the spring element can be inserted in order to secure the resistance body (23) in a certain angle position.

5. A therapy device (1) for supporting the respiration and expectoration of a patient by means of which an oscillating air resistance can be produced in the air flow during exhalation and inhalation, comprising a mouthpiece (2) having an inlet opening (7) provided on one of its ends and an elastically deformable hose section (3) attached to its end that is opposite to the inlet opening (7), and a housing (10) that is firmly connected to the mouthpiece (2) and by means of which the hose section (3) is enclosed wholly or partially in the circumferential direction,
**characterised in that,**
a resistance body (23) articulated to allow it to move on the housing (10) is arranged at a distance from the mouthpiece (2) inside the housing (10), that the resistance body (23) is provided as a support for one section (5) of the hose section (3) and that the support allows the curvature of the hose section (3) to be adjusted.

6. The therapy device in accordance with Claims 5,
**characterised in that,**
the hose section (3), preferably the free end of the hose section (3), can be moved during inhalation and exhalation in an oscillating fashion between the resistance body (23) and the inner wall of the housing (10) that is opposite to the resistance body (10).

7. The therapy device in accordance with one or more of the aforementioned claims,
**characterised in that,**
the mouthpiece (2) together with the hose section (3) can be aligned so as to move in the longitudinal axis (9) relative to the resistance body (23).

8. The therapy device in accordance with one or more of the aforementioned claims,
**characterised in that,**
the resistance body (23) is configured as a plate and that a section (5) of the hose section (3) can be moved by the resistance body (23) in the unactuated condition to a height level that is located below or above the section (4) of the hose section (3) attached to the mouthpiece (2).

9. The therapy device in accordance with one or more of the aforementioned claims,
**characterised in that,**
the hose section (3) can be aligned in differently angled positions in relation to the resistance body (23).

10. The therapy device in accordance Claim 5,
**characterised in that,**
the housing (10) is configured fully enclosed, that an opening (31) is worked into one of the side walls of the housing (10) and that a second mouthpiece (2') is inserted in the opening (31).

## Revendications

1. Appareil de thérapie (1) servant à assister la respiration, le dégagement de mucus et l'expectoration d'un patient, et qui, lors de l'inspiration et de l'expiration, crée une résistance d'air oscillante, avec un embout (2) dont une face frontale comporte une ouverture d'entrée (7) et dont la face frontale opposée à l'ouverture d'entrée porte un tuyau flexible déformable (3),
**caractérisé en ce que**,
sur l'embout (2), il est prévu une plaque de portée (21) qui, au niveau de l'embout (2) est dirigée parallèlement au tuyau flexible déformable (3), que sur l'extrémité libre de la plaque de portée (21) il est prévu une articulation (22) par laquelle un corps de résistance (23) est articulé sur la plaque de portée (21) et que le corps de résistance (23) collabore avec la partie lui assignée du tuyau flexible (3) de sorte que la section de passage du tuyau flexible (3) se laisse ajuster de l'extérieur.

2. Appareil de thérapie d'après la revendication 1,
**caractérisé en ce que**,
en direction du tuyau flexible (3) et référé à l'axe longitudinal (9) de l'appareil de thérapie (1), le corps de résistance (23) se laisse dévier d'entre 0° et 50°.

3. Appareil de thérapie d'après les revendications 1 ou 2,
**caractérisé en ce que**,
l'embout (2) est inséré dans un boîtier (10) qui renferme le tuyau flexible (3) en direction du pourtour, que l'extrémité du boîtier (10) opposée à l'embout (2) possède une ouverture de passage sous la forme d'une trompète ou d'un V et que le corps de résistance (23) est disposé au niveau de l'ouverture de passage.

4. Appareil de thérapie d'après la revendication 3,
**caractérisé en ce que**,
sur le corps de résistance (23), il est prévu un nez de crantage (24), un élément élastique ou une pièce similaire, saillant en direction d'une des deux parois latérales du boîtier (10) et que dans la paroi du boîtier, il est pratiqué un ou plusieurs perçages (25), dans lesquels le nez de crantage (24) ou l'élément élastique se laissent insérer afin de positionner le corps de résistance (23) sous un certain angle.

5. Appareil de thérapie (1) servant à assister la respiration et l'expectoration d'un patient, et qui, lors de l'inspiration et de l'expiration, crée une résistance d'air oscillante, avec un embout (2) dont une face frontale comporte une ouverture d'entrée (7) et dont la face frontale opposée à l'ouverture d'entrée (7) porte un tuyau flexible déformable (3), et d'un boîtier (10) lié rigidement à l'embout (2) et qui renferme le tuyau flexible (3) entièrement ou partiellement en direction du pourtour,
**caractérisé en ce que**,
à une certaine distance de l'embout (2) il est prévu à l'intérieur du boîtier (10) un corps de résistance (23) articulé dans celui-ci, que le corps de résistance (23) est conçu sous la forme d'un support pour une section (5) du tuyau flexible (3) et que, par l'intermédiaire du support, il est possible de régler la courbure du tuyau flexible (3).

6. Appareil de thérapie d'après la revendication 5,
**caractérisé en ce que**,
lors de l'aspiration et de l'expiration, le tuyau flexible (3), en particulier l'extrémité libre du tuyau flexible (3), puisse osciller entre le corps de résistance (23) et la paroi intérieure du boîtier (10) qui lui est opposée.

7. Appareil de thérapie d'après une ou plusieurs des revendications précédentes,
**caractérisé en ce que**,
dans l'axe longitudinal (9), l'embout (2) avec le tuyau flexible (3) se laissent ajuster de manière mobile par rapport au corps de résistance (23).

8. Appareil de thérapie d'après une ou plusieurs des revendications précédentes,
**caractérisé en ce que**,
le corps de résistance (23) est conçu sous la forme d'une plaque et qu'en état désactivé, une section (5) du tuyau flexible (3) se laisse monter depuis le corps de résistance (23) à un niveau qui se trouve au-dessus ou en dessous de la section (4) du tuyau flexible (3) fixée sur l'embout (2).

9. Appareil de thérapie d'après une ou plusieurs des revendications précédentes,
**caractérisé en ce que**,
par rapport au corps de résistance (23), le tuyau flexible (3) se laisse régler dans différentes positions angulaires.

10. Appareil de thérapie d'après la revendication 5,
**caractérisé en ce que**,
le boîtier (10) est entièrement fermé, que dans une des parois latérales du boîtier (10), il est prévu une ouverture (31) et qu'un deuxième embout (2') est inséré dans l'ouverture (31).
